# EUROPEAN PATENT APPLICATION

(11) **EP 4 560 643 A1**
(43) Date of publication of application: **28.05.2025**
(21) Application number: 23211672.3
(22) Date of filing: 23.11.2023
(51) Int. Cl.: G16H 30/20

(54) **MEANS FOR MANAGING A DENTAL ALIGNER TREATMENT IN DEPENDENCE OF MAGNETIC RESONANCE DATA**

(71) Applicant: Siemens Healthineers AG, 91301 Forchheim (DE); Sirona Dental Systems GmbH, 64625 Bensheim (DE); Dentsply Sirona Inc., York, PA 17401 (US)
(72) Inventor: Burzan, Kim, 69488 Birkenau (DE); Ulrici, Johannes, 64285 Darmstadt (DE); Greiser, Andreas, 91054 Erlangen (DE); Lauer, Lars, 91077 Neunkirchen (DE)
(74) Representative: Siemens Healthineers Patent Attorneys

(57) **Abstract**

The present invention relates to a system (1) for managing a dental aligner treatment of a patient (2), the system (1) comprising an acquisition device (3; 3a; 3b) adapted to acquire magnetic resonance data (4) of a maxillofacial region of the patient (2), and impression data (5) of at least some of the teeth (6) of the patient (2); and a computing device (7) configured to analyse the magnetic resonance data (4) and determine a condition of a the maxillofacial region of the patient (2). The invention further relates to a computer-implemented method for managing a dental aligner treatment of a patient (2) using an inventive system (1). Furthermore, the invention relates to a computer program product comprising program code means to perform an inventive method when the computer program product is executed in a processing unit of an inventive system (1).

## Description

Independent of the grammatical term usage, individuals with male, female or other gender identities are included within the term.

The present invention relates to the orthodontic treatment of tooth misalignments of a patient by using individually manufactured dental aligners. The present invention more particularly relates to a system for managing a dental aligner treatment of a patient.

Orthodontic treatments are usually carried out according to the following general steps:
1. During an initial consultation, an orthodontist examines the patient's teeth, jaw, and bite and reviews the patient's dental and medical history. Subsequently, the orthodontist determines the treatment goals.
2. The orthodontist typically takes a series of diagnostic records, including photographs, x-ray images, and digital impressions of the teeth. These records help the orthodontist to visualize the patient's teeth and jaw in three dimensions and enable the orthodontist to diagnose any problems accurately.
3. Based on the patient's examination and diagnostic records, the orthodontist develops a diagnosis and a treatment plan. This treatment plan includes specific recommendations for appliances, such as braces or dental aligners, but also the duration of the treatment.
4. During the dental aligner treatment, the patient typically wears a plurality of dental aligners that have been created in accordance with the treatment plan. The dental aligners may have to be used in a specific sequence to achieve the treatment goal. The duration of the dental aligner treatment depends on the complexity of the treatment goal, but it typically ranges from 6 months to 2 years or more.
5. After the initial phase of the dental aligner treatment is complete, the patient may need to wear a retainer to maintain the alignment of the teeth. The orthodontist schedules periodic follow-up appointments to monitor the patient's progress and make any necessary adjustments to the treatment plan.

A problem is that a detection of treatment-induced issues such as inflammation and/or root resorption, and a consequent adaptation of the dental aligner treatment to avoid posttreatment health issues is often not possible due to limited diagnostic means. Besides a limited ability to identify treatment-induced issues using x-ray imaging methods during the course of a treatment, a collection of x-ray data during the treatment for monitoring purposes exposes the patient to ionizing radiation and is typically not indicated, while a simple visual inspection of the oral cavity by the dentist provides only limited insight into health issues beneath the visible surface.

Even if treatment-induced issues could be detected with means available in a dental facility, the orthodontist may not be able to specify how the treatment should be adapted to prevent or correct such issues.

This is also true when the treatment involves a use of dental aligners, in particular clear aligners.

The treatment with aligners, in particular clear aligners, is usually based on information resulting from digital impression (DI) scans, photos, and optionally x-ray images.

Typically, an aligner design is planned based on an initial DI scan ("default" case), i. e. without considering an individual patient condition and without means for optimizations during the treatment. This may lead to damage to the patient, such as a loss of teeth due to too rapid teeth movement and subsequent inflammations, a shortening of roots of the teeth, root resorption, and/or problems in the temporomandibular joint (TMJ) region, resulting in the requirement of further treatment after initial treatment has been completed.

At present, the design of clear aligners is based on a visual inspection and a DI scan, photographs, and/or a panoramic x-ray scan. In some cases, the acquisition of a 3D cone beam CT (computed tomography) image is recommended.

Orthodontic brackets are usually designed based on a cephalometric x-ray image (Ceph scan). The Ceph scan can also be used for treatment planning, for example via identification of anatomical landmarks.

Furthermore, a PAN scan (i. e. a whole-body CT scan) can be used to identify locations of the teeth and the roots of the patient. However, information from Ceph scans or PAN scans is rarely used for treatment planning or monitoring when using clear aligners.

The acquisition of an x-ray and/or a CT image exposes the patient to ionizing radiation. Ionizing radiation can cause immediate radiation damage to tissue cells in irradiated areas of the patient's body and has a potential risk of altering a cell's genetic makeup which may lead to the development of tumours and cancer. Thus, imaging modalities using ionizing radiation are rarely used to support planning or monitoring of a dental aligner treatment.

### SUMMARY OF THE INVENTION

An objective of the present invention is to provide an adapted dental aligner treatment of a patient which reduces a need for follow-up treatments of dental aligner treatment induced issues.

This objective is achieved by the system and the computer-implemented method according to the independent claims. The subject-matters of the dependent claims define further developments and preferred embodiments.

The system of the present invention is configured for managing a dental aligner treatment of a patient.

The system comprises an acquisition device adapted to acquire magnetic resonance data of a maxillofacial region of the patient, and impression data of a plurality of teeth of the patient.

Furthermore, the system comprises a computing device adapted to:
- determine treatment information in dependence of the impression data, wherein the treatment information comprises a design of one or more dental aligners to be used during the dental aligner treatment of the patient and/or an initial schedule for an exchange of the designed dental aligners during the dental aligner treatment of the patient,
- output an initial dental aligner treatment plan proposal comprising the treatment information,
- analyse the magnetic resonance data and determine a condition of the maxillofacial region of the patient, and
- propose a modification to the initial dental aligner treatment plan proposal in dependence of the determined condition of the maxillofacial region of the patient.

The computing device may comprise a computing unit or a processing unit configured to process the magnetic resonance data and the impression data. Particularly, the processing unit may be configured to extract information according to an embodiment described below from the magnetic resonance data and the impression data. In some embodiments, the computing device may correspond to the processing unit.

In a preferred embodiment, the computing device comprises a control unit configured to control the acquisition device to acquire the magnetic resonance data of a maxillofacial region of the patient, and the impression data of a plurality of teeth of the patient. The control unit may also be configured to control the processing unit to process the magnetic resonance data and the impression data.

The maxillofacial region may comprise at least a tooth, a part of a jaw, a part of a dental arch, a part of the mouth, a part of a jawbone, and/or a part of a craniomaxillofacial complex of the patient.

In a preferred embodiment, the magnetic resonance data comprises one or more magnetic resonance images. The computing device may be configured to analyse the one or more magnetic resonance images and determine the condition of the maxillofacial region of the patient in dependence of the one or more magnetic resonance images. The magnetic resonance data may also comprise magnetic resonance signals and/or k-space data of the maxillofacial region of the patient.

According to an embodiment, the magnetic resonance data is acquired before the one or more dental aligners are manufactured.

In a preferred embodiment, the magnetic resonance data is acquired after the one or more dental aligners have been manufactured. Acquiring magnetic resonance data after the treatment has started may favourably allow for a detection of undesired effects of the dental aligner treatment on the maxillofacial region of the patient, such as inflammation, bone resorption, tooth root resorption, and the like. For example, the magnetic resonance data may favourably be used in longitudinal imaging studies (i. e. by continuously monitoring the maxillofacial region of the patient over the course of the dental aligner treatment), which is not feasible with imaging modalities based on ionizing radiation due to accumulating radiation doses, but also low soft tissue contrast and poor visibility of inflammation.

The system may comprise a plurality of acquisition devices adapted to acquire one or more magnetic resonance images of the maxillofacial region of the patient, and impression data of a plurality of teeth of the patient. For example, the system may comprise a first acquisition device configured to acquire or receive magnetic resonance data of the maxillofacial region of the patient, and a second acquisition device configured to acquire or receive impression data of a plurality of teeth of the patient.

The acquisition device may comprise a magnetic resonance device configured to acquire the magnetic resonance data of the maxillofacial region of the patient via a magnetic resonance examination. However, the acquisition device may also comprise a data interface configured to receive the magnetic resonance data of the maxillofacial region of the patient from a magnetic resonance device, a network system (e. g. a PACS, a RIS, or a HIS), a cloud, and/or a data storage. According to an embodiment, the acquisition device comprises a control unit configured to control a magnetic resonance device to acquire or receive the magnetic resonance data of the maxillofacial region of the patient.

In a preferred embodiment, the magnetic resonance data comprises information on internal structures of the maxillofacial region of the patient. For example, the magnetic resonance data may comprise information on an internal structure or composition of a tooth, a plurality of teeth, a dental arch, a jawbone, a mandibular joint, a section of the gingiva, and the likes.

The acquisition device may comprise a scanning device configured to acquire impression data of the plurality of teeth of the patient. It is also conceivable that the acquisition device comprises a data interface configured to acquire the impression data from a scanning device, from a network system (e. g. a PACS, a RIS, or a HIS), a cloud, and/or a data repository.

Outputting the initial dental aligner treatment plan proposal may comprise storing the initial dental aligner treatment plan proposal in a data storage, e. g. a local data storage, a network data storage, and/or a cloud data storage. However, outputting the initial dental aligner treatment plan proposal may also comprise transmitting the initial dental aligner treatment plan proposal to a display unit, a treatment planning unit, and/or a dental aligner manufacturing apparatus. Particularly, outputting the initial dental aligner treatment plan proposal may comprise outputting the initial dental aligner treatment plan proposal to a practitioner, e. g. an orthodontist, a dental professional, a dentist, and/or a dental technician.

The output initial dental aligner treatment plan proposal may comprise the proposed modification to the initial dental aligner treatment plan proposal.

The inventive system for managing a dental aligner treatment of a patient allows for a modification of the dental aligner treatment in dependence of magnetic resonance data of the maxillofacial region of a patient. The magnetic resonance data may comprise information on internal structures of the maxillofacial region of the patient favourably complementing information from other sources, such as the impression data. Thus, the dental aligner treatment may favourably be improved and/or tailored to a specific patient in dependence of the information on the internal structures of the maxillofacial region of the patient.

The inventive system may favourably allow for a reduction of a requirement of post treatment of aligner treatment-induced issues. In providing a proposed modification to the initial dental aligner treatment plan proposal, a risk of damage to the patient due to the dental aligner treatment can favourably be reduced or minimized.

In using magnetic resonance data, an exposure of the patient to ionizing radiation can favourably be avoided. Thus, a progress of the dental aligner treatment can be monitored continuously or at specific times over the course of the dental aligner treatment, e. g. in a longitudinal imaging series.

The inventive system may be particularly beneficial in medical facilities without access to a magnetic resonance device. The inventive system may favourably allow for an acquisition of the magnetic resonance data and a modification of the initial dental aligner treatment plan proposal after the initial dental aligner treatment plan proposal has been established based on the impression data.

According to an embodiment of the inventive system, the computing device is adapted to output the proposed modification to a practitioner.

As described above, the computing device is configured to output the initial dental aligner treatment plan proposal. The initial dental aligner treatment plan proposal may include the proposed modification to the initial dental aligner treatment plan proposal. However, the proposed modification to the initial dental aligner treatment plan proposal and the initial dental aligner treatment plan proposal may be output separately. In a preferred embodiment, the proposed modification to the initial dental aligner treatment plan proposal and/or the initial dental aligner treatment plan proposal are output to the practitioner via a display unit, such as a screen, a monitor, a projector, a screen of mobile device, or the like.

In outputting the proposed modification to the practitioner, the practitioner may favourably be made aware of necessary modifications to the dental aligner treatment.

According to a further embodiment of the inventive system, the proposed modification to the initial dental aligner treatment plan proposal comprises a heat map and/or a symbol indicative of an anatomical region for which the dental aligner treatment should be adapted.

Preferably, the computing device comprises an image processing unit configured to highlight and/or annotate the anatomical region for which the dental aligner treatment should be adapted. Particularly, the image processing unit may be configured to highlight the anatomical region for which the dental aligner treatment should be adapted in a magnetic resonance image, a representation of a set of teeth of the patient, a panoramic view of a set of teeth of the patient, and/or a physical or virtual model of the set of teeth of the patient.

A heat map may indicate the anatomical region for which the dental aligner treatment should be adapted in a different colour. A symbol may comprise a letter, a numeral, a mark, a sign, a graphic, or the like. Preferably, the heat map and/or the symbol are configured to provide an indication how the anatomical region should be adapted. For example, the heat map and/or the symbol may provide an indication how a shape and/or dimension of the one or more dental aligners should be modified.

In providing a heat map and/or a symbol indicative of an anatomical region for which the dental aligner treatment should be adapted, a risk of misinterpretation of the proposed modification and/or damage to the patient may favourably be reduced or eliminated.

According to an embodiment of the inventive system, the treatment information comprises a design of one or more dental aligners to be used during the dental aligner treatment of the patient and the proposed modification to the initial dental aligner treatment plan proposal comprises a modification to the design of the one or more dental aligners.

The design of the one or more dental aligners may comprise information on a dimension and/or a shape of the one or more dental aligners. For example, the design of the one or more dental aligners may comprise three-dimensional information on the one or more dental aligners. In a preferred embodiment, the design of the one or more dental aligners comprises a three-dimensional model of each dental aligner of the one or more dental aligners.

It is conceivable that the proposed modification to the initial dental aligner treatment plan proposal comprises a modification to a dimension and/or a shape of a dental aligner of the one or more dental aligners. Particularly, the proposed modification to the initial dental aligner treatment plan proposal may comprise a modification or a change of at least a portion of the three-dimensional model of a dental aligner or a plurality of dental aligners of the one or more dental aligners.

According to an embodiment, the proposed modification to the initial dental aligner treatment plan proposal comprises a modification to the initial schedule for the exchange of the designed dental aligners during the dental aligner treatment of the patient.

In providing modifications to a design of one or more dental aligners in dependence of an assessment of a condition of a maxillofacial region of a patient based on magnetic resonance data, a quality of the dental aligner treatment may favourably be improved in comparison to conventional dental aligner treatments.

In a further embodiment of the inventive system, the computing device is adapted to output the proposed modification to the design of the one or more dental aligners to a manufacturing apparatus.

A manufacturing apparatus may comprise an additive manufacturing apparatus and/or a machining manufacturing device. In a preferred embodiment, the manufacturing apparatus comprises an additive manufacturing apparatus configured to manufacture the one or more dental aligners via a 3D printing process. A 3D printing process may comprise an additive manufacturing technique, such as vat photopolymerization, a material jetting, a binder jetting, a powder bed fusion, a material extrusion, a directed energy deposition, a sheet lamination, or the like.

The proposed modification to the design of the one or more dental aligners may favourably be transmitted or output directly to a manufacturing apparatus configured to manufacture the one or more dental aligners. Thus, a process efficiency of implementing required adaptations to the dental aligner treatment based on the condition of the maxillofacial region of the patient may favourably be improved.

According to an embodiment, the inventive system comprises a scanning device for generating the impression data by scanning an oral cavity of the patient and/or by scanning impression material which has been applied to the teeth of the patient.

The scanning device may be configured to scan a 3D surface of an intraoral region of the patient and/or the impression material. Particularly, the scanning device may be configured to acquire 3D information and/or a 3D scan of the intraoral region or oral cavity of the patient and/or the impression material. According to an embodiment, the scanning device is an intraoral scanning device configured to acquire 3D information of the intraoral region or oral cavity of the patient.

The oral cavity or intraoral region of the patient may comprise a tooth, a plurality of teeth, a dental arch, a section of a gingiva, a section of the tongue, a section of the palate, a section of the dentition, or the like.

The scanning device may comprise one or more cameras. For example, the scanning device may comprise a plurality of 2D cameras and/or a 3D camera. It is also conceivable that the scanning device comprises an infrared camera and/or a LIDAR sensor.

Inserting a dental impression material into an oral cavity of a patient may be repulsive to some patients. In providing an inventive system comprising a scanning device, a risk of termination of a process of taking a dental impression by the patient may favourably be reduced.

In providing an inventive system comprising a scanning device, a process efficiency of acquiring impression data of a plurality of teeth of a patient may favourably be improved.

According to a further embodiment of the inventive system, the computing device is adapted to analyse the impression data to detect a pathology in the maxillofacial region of the patient and propose a modification to the initial dental aligner treatment plan proposal in dependence of the determined pathology of the maxillofacial region of the patient.

The impression data may comprise 3D information on the plurality of teeth of the patient. Particularly, the impression data may comprise a 3D model of the plurality of teeth of the patient. In a preferred embodiment, the impression data comprises a 3D scan of the plurality of teeth of the patient and/or a 3D of an impression material which has been applied to the teeth of the patient.

The computing device may comprise a processing unit configured to analyse the impression data and detect a pathology in the maxillofacial region of the patient. Particularly, the computing device may comprise an image processing unit configured to process a 3D scan of the plurality of teeth of the patient and/or an impression material which has been applied to the teeth of the patient and detect a pathology in the maxillofacial region of the patient in dependence of the 3D scan.

For example, the processing unit may comprise an AI algorithm, particularly a trained AI algorithm, configured to process the impression data and detect a pathology in the maxillofacial region of the patient. The processing unit may also be configured to detect a pathology in the maxillofacial region of the patient based on impression data from one or more reference patients. The processing unit may be configured to acquire the impression data from one or more reference patients from a data storage, e. g. a local data storage, a network storage, and/or a cloud storage. Furthermore, the processing unit may be configured to detect a pathology in the maxillofacial region of the patient in dependence of a model, e. g. a body model, particularly a model of the maxillofacial region. The processing unit may be configured to detect the pathology in the maxillofacial region of the patient in dependence of an arrangement of teeth, an orientation of at least one tooth, a position of at least one tooth, a relative position a plurality of teeth, a missing tooth, a missing part of at least one tooth, and/or a damage to at least one tooth.

It is conceivable that the computing device comprises a processing unit configured to propose a modification to the initial dental aligner treatment plan proposal in dependence of the determined pathology of the maxillofacial region of the patient.

A processing unit may correspond to a local processor of the computing device. However, the processing unit may also form a component of a network system and/or a cloud processor.

A computing device according to the invention may favourably allow for consistent and/or reproduceable results regarding proposed modifications to the dental aligner treatment.

Furthermore, a computing device according to the invention may favourably allow for a detection of a pathology in the maxillofacial region of the patient regardless of a qualification of the practitioner or dental professional.

According to an embodiment of the inventive system, the acquisition device is configured to acquire further magnetic resonance data of the maxillofacial region of the patient. The computing device is configured to:
- determine further treatment information in dependence of the further magnetic resonance data, and
- output the further treatment information.

The computing device may be configured to arrange for a further magnetic resonance examination to be carried out during the dental aligner treatment of the patient. For example, the computing device may be configured to notify a dental professional that further magnetic resonance data of the maxillofacial region of the patient should be acquired.

The computing device may be configured to prescribe and/or initiate the further magnetic resonance examination of the maxillofacial region of the patient at any time during the dental aligner treatment.

The further magnetic resonance data differs from the magnetic resonance data. Preferably, the further magnetic resonance data is acquired after the magnetic resonance data has been acquired. Particularly, the further magnetic resonance data may be acquired after the dental aligner treatment has started, e. g. after one or more dental aligners based on the initial dental aligner treatment plan proposal and/or the proposed modification to the initial dental aligner treatment plan proposal have been applied to the teeth of the patient.

The computing device may comprise a processing unit and/or an image processing unit according to an embodiment described above. The processing unit and/or image processing unit may be configured to analyse the further magnetic resonance data and determine further treatment information in dependence of the further magnetic resonance data.

Furthermore, computing device may be configured to determine a further dental aligner treatment plan proposal comprising the further treatment information. Preferably, the further dental aligner treatment plan proposal differs from the dental aligner treatment plan proposal. It is also conceivable that the further treatment information comprises a proposed modification to the initial dental aligner treatment plan proposal and/or the proposed modification to the dental aligner treatment plan proposal according to an embodiment described above.

The computing device may be configured to output the further dental aligner treatment plan proposal according to an embodiment described above. Preferably, the computing device is configured to output the further treatment information to a data storage, a network system, a cloud, and/or a practitioner.

In determining further treatment information, the dental aligner treatment may favourably be modified or adapted in dependence of changes to the condition of the maxillofacial region of the patient during the dental aligner treatment.

In a preferred embodiment of the inventive system, the computing device is configured to:
- determine a pathology in the maxillofacial region of the patient in dependence of the magnetic resonance data, and
- propose a modification to the initial dental aligner treatment plan proposal in dependence of the determined pathology of the maxillofacial region of the patient.

The computing device may comprise a processing unit and/or an image processing unit according to an embodiment described above. The computing device may be configured to process the magnetic resonance data and determine a pathology in the maxillofacial region of the patient in dependence of the magnetic resonance data. Furthermore, the computing device may be configured to determine a modification to the initial dental aligner treatment plan proposal in dependence of the determined pathology of the maxillofacial region of the patient.

The pathology in the maxillofacial region of the patient may comprise, for example, an inflammation, an inflammation at a tooth root tip, a bone resorption, a tooth root resorption, a change in a pulp or bone perfusion, and/or a deviation from an expected course of the dental aligner treatment.

A computing device according to the invention may favourably allow for a detection of a pathology in the maxillofacial region of the patient regardless of a qualification of the practitioner or dental professional. For example, some dental professionals may be unfamiliar with a correct functioning of temporomandibular joints. In providing a computing device configured to propose a modification to the initial dental aligner treatment plan proposal in dependence of the determined pathology of the maxillofacial region of the patient, a quality of the dental aligner treatment may be improved. Furthermore, a risk of damage to the patient via an inadequate dental aligner treatment (e. g. based on insufficient information from impression data) may favourably be reduced or eliminated.

According to an embodiment of the inventive system, the computing device is configured to:
- determine a deviation between an arrangement of teeth according to the initial dental aligner treatment plan proposal and a physiologically appropriate arrangement of teeth in dependence of the magnetic resonance data, and
- propose a modification to the initial dental aligner treatment plan proposal in dependence of the determined deviation.

An arrangement of teeth according to the initial dental aligner treatment plan proposal may correspond to an arrangement of teeth obtained when the dental aligner treatment is completed without making modifications to the initial dental aligner treatment plan proposal.

A physiologically appropriate arrangement of teeth may correspond to an arrangement of teeth providing a healthy bite (e. g. characterized by a correct position of an upper jaw in relation to a lower jaw) and/or allowing for a correct functioning of the temporomandibular joints of the patient.

The computing device may comprise a processing unit and/or an image processing unit according to an embodiment described above. The computing device may be configured to determine an arrangement of teeth according to the initial dental aligner treatment plan proposal, but also a physiologically appropriate arrangement of teeth. The computing device may be configured to determine the physiologically appropriate arrangement of teeth in dependence of the magnetic resonance data of the maxillofacial region of the patient. It is also conceivable, that the computing device is configured to determine the physiologically appropriate arrangement of teeth in dependence of data from reference patients and/or a model, particularly a model comprising a maxillofacial region.

According to an embodiment of the inventive system, the magnetic resonance data comprises magnetic resonance data of a temporomandibular joint of the patient, and the computing device comprises a motion simulator configured to simulate a motion of a temporomandibular joint in dependence of the magnetic resonance data.

The magnetic resonance data may comprise information on a dimension and/or a shape of a temporomandibular joint of the patient. Preferably, the magnetic resonance data comprises 3D information, particularly 3D image data, of a temporomandibular joint of the patient.

The motion simulator may be a part of a processing unit of the computing device. It is also conceivable that the motion simulator is implemented on a processing unit of the computing device, a network system and/or a cloud. The motion simulator may comprise a model of the maxillofacial region, particularly a model of a temporomandibular joint.

The motion simulator may be configured to determine a motion of the temporomandibular joint in dependence of the magnetic resonance data of the temporomandibular joint of the patient. Preferably, the motion simulator is configured to emulate or simulate a motion of the temporomandibular joint in dependence of an arrangement of teeth, e. g. the arrangement of teeth according to the initial dental aligner treatment plan proposal, but also the physiologically appropriate arrangement of teeth. It is also conceivable that the motion simulator is configured to generate or adapt a model comprising the maxillofacial region in dependence of the magnetic resonance data. The motion simulator may be configured to simulate a motion of a temporomandibular joint using the model.

According to an embodiment, magnetic resonance data and/or the further magnetic resonance data comprise image data of the maxillofacial region of the patient with different opening and/or closing positions of the jaw or temporomandibular joint. The motion simulator may be configured to output or display an animation of the motion of the jaw or temporomandibular joint based on the magnetic resonance data and/or the further magnetic resonance data.

An inventive system may favourably allow for an adaption of the dental aligner treatment in dependence of a functionality of the temporomandibular joint. Thus, a risk of damaging the patient via the dental aligner treatment may favourably be reduced or eliminated.

The inventive computer-implemented method for managing a dental aligner treatment of a patient comprises the steps:
- acquiring magnetic resonance data of a maxillofacial region of the patient and impression data of a plurality of teeth of the patient via an acquisition device,
- determine treatment information in dependence of the impression data via a computing device, wherein the treatment information comprises a design of one or more dental aligners to be used during the dental aligner treatment of the patient and/or an initial schedule for the exchange of the designed dental aligners during the dental aligner treatment of the patient,
- output an initial dental aligner treatment plan proposal comprising the treatment information,
- analyse the magnetic resonance data and determine a condition of the maxillofacial region of the patient, and
- propose a modification to the initial dental aligner treatment plan proposal in dependence of the determined condition of the maxillofacial region of the patient.

The inventive method may be carried out using an inventive system according to an embodiment described above. Particularly, the inventive method may be configured to carry out each function of an inventive system according to an embodiment described above. Preferably, the inventive system according to an embodiment described above is configured to carry out the inventive method.

It is to be understood that all features, functions, and embodiments of an inventive system described herein also apply to an inventive method or can be combined with an inventive method unless this is expressly excluded.

The inventive system may comprise a magnetic resonance device, a scanning device, a control unit, and/or a processing unit. The control unit may be configured to control the computing device to perform any of the method steps and/or functions of an embodiment of an inventive system described above.

The magnetic resonance data of a maxillofacial region of the patient may be acquired before or after the dental aligner treatment has started. For example, the magnetic resonance data and the impression data may be acquired substantially at the same time (e. g. on the same day or in the same week). However, the magnetic resonance data may also be acquired after the dental aligner treatment has started. In particular, the magnetic resonance data may be acquired during the dental aligner treatment. It is conceivable that magnetic resonance data is acquired multiple times over the course of the dental aligner treatment. In this case, one or more steps of the inventive method may be repeated multiple times.

According to an embodiment, the control unit of the inventive system is configured to control the magnetic resonance device to acquire magnetic resonance data from a maxillofacial region of a patient. It is also conceivable that the control unit is configured to control the optical imaging device to acquire optical data or impression data from the oral cavity of the patient and/or an impression material which has been applied to the teeth of the patient.

The computing device may represent a main computing device of a magnetic resonance device and/or a scanning device. However, the computing device may also represent an independent or standalone component. Likewise, the control unit of the inventive system may correspond to a main control unit of a magnetic resonance device and/or a main control unit of a scanning device. In case the computing device of the inventive system forms a standalone component, the main control unit of the inventive system may be connected to the main control unit of the magnetic resonance device and/or the main control unit of the scanning device via a suitable signal connection.

The inventive system, particularly the computing device, may comprise one or more acquisition devices or data interfaces configured to receive or acquire the impression data and the magnetic resonance data of the maxillofacial region of the patient. For example, the computing device may comprise one or more data interfaces connected to the scanning device and/or the magnetic resonance device via a suitable signal connection (i. e. an electrical signal connection or a wireless connection). It is also conceivable, however, that the one or more data interfaces are connected to a data storage, a network system, or a cloud according to an embodiment described above.

In some embodiments, the processing unit of the computing device may represent a main processing unit of the magnetic resonance device and/or a main processing unit of a scanning device. In case the computing device is an independent component, the processing unit may be connected to the main processing unit of the magnetic resonance device and/or the main processing unit of the scanning device via a suitable signal connection. It is conceivable that the processing unit is incorporated or integrated within the control unit.

Preferably, the processing unit and the control unit of the computing device are configured to coordinate and/or carry out the inventive method and each function of an embodiment of the inventive system according to an embodiment described above.

The processing unit and/or the control unit may form a part of the computing device. The processing unit and/or the control unit may comprise a graphics unit, a storage unit and any further component required for carrying out the inventive method.

According to an embodiment, the processing unit is configured to process the impression data and/or the magnetic resonance data. Particularly, the processing unit may be configured to analyse the magnetic resonance data and/or the impression data and determine a condition of the maxillofacial region of the patient. The processing unit may comprise or implement a processing algorithm, such as an image processing algorithm, a pattern recognition algorithm, or the like. The processing algorithm may be configured to process the impression data and/or the magnetic resonance data according to an embodiment described above.

The inventive method shares the advantages of the inventive system. The inventive system may favourably allow for carrying out an embodiment of an inventive method in a robust and/or reproduceable manner.

The inventive system for managing a dental aligner treatment of a patient comprises an acquisition device adapted to acquire magnetic resonance data of a maxillofacial region of the patient, and impression data of a plurality of teeth of the patient. The system further comprises a computing device configured to:
- analyse the magnetic resonance data and determine a condition of the maxillofacial region of the patient,
- determine treatment information in dependence of the impression data and the determined condition of the maxillofacial region of the patient (2), wherein the treatment information comprises a design of one or more dental aligners to be used during the dental aligner treatment of the patient and/or an initial schedule for an exchange of the designed dental aligners during the dental aligner treatment of the patient,
- output a dental aligner treatment plan proposal comprising the treatment information.

The system may be configured according to an embodiment described above. Particularly, the system may comprise and/or implement any of the components and/or functions of an inventive system according to an embodiment described above. Preferably, the acquisition device and/or the computing device of the system are embodied according to an embodiment of the system described above.

In contrast to an embodiment of the system described above, the computing device is configured to directly generate a dental aligner treatment plan proposal in dependence of the magnetic resonance data of the maxillofacial region of the patient, rather than determining a modification to an initial dental aligner treatment plan proposal which is based on the impression data.

In providing an inventive system, an efficiency of generating a dental aligner treatment plan proposal may favourably be increased. The system may be particularly beneficial in medical facilities comprising a magnetic resonance device. Such facilities may allow for an acquisition of the impression data and the magnetic resonance data substantially at the same time. Thus, the dental aligner treatment plan proposal can be generated immediately after acquisition of the impression data and the magnetic resonance data.

The inventive computer-implemented method for managing a dental aligner treatment of a patient comprises the steps:
- acquiring magnetic resonance data of a maxillofacial region of the patient and impression data of a plurality of teeth of the patient via an acquisition device,
- analysing the magnetic resonance data and determining a condition of the maxillofacial region of the patient via a computing device,
- determining treatment information in dependence of the impression data and the determined condition of the maxillofacial region of the patient via the computing device, wherein the treatment information comprises a design of one or more dental aligners to be used during the dental aligner treatment of the patient and/or an initial schedule for an exchange of the designed dental aligners during the dental aligner treatment of the patient, and
- outputting a dental aligner treatment plan proposal comprising the treatment information.

The inventive method may be carried out using an inventive system according to an embodiment described above. Particularly, the inventive method may be configured to carry out each function of an inventive system according to an embodiment described above. Preferably, the inventive system according to an embodiment described above is configured to carry out the inventive method.

It is to be understood that all features, functions, and embodiments of an inventive system described herein also apply to an inventive method or can be combined with an inventive method unless this is expressly excluded.

In determining treatment information in dependence of the impression data and the determined condition of the maxillofacial region of the patient, the dental aligner treatment plan proposal may favourably consider information on internal structures of the maxillofacial region of the patient extracted from the magnetic resonance data.

The inventive method shares the advantages of the inventive system. The inventive system may favourably allow for carrying out an embodiment of an inventive method in a robust and/or reproduceable manner.

The inventive computer program product can be loaded into a memory of a programmable processing unit of an inventive system according to an embodiment described above. The computer program product comprises program code means to perform an inventive method when the computer program product is executed in the processing unit of the inventive system.

As a result, the inventive method can be carried out quickly, and in a robust and repeatable manner. The computer program product is configured in such a way that it can carry out the method steps and functions according to the invention by means of the computing device. The computing device must in each case comprise the prerequisites, such as a corresponding processing unit, a corresponding main memory, a corresponding graphics card, or a corresponding control unit, so that the respective method steps and functions can be carried out efficiently.

The computer program product is, for example, stored on a computer-readable medium or stored on a network, a server, or a cloud, from where it can be loaded into the processing unit of the computing device. The processing unit can be connected to the computing device or form a part of the computing device.

Control information of the computer program product can be stored on an electronically readable medium. The control information on the electronically readable medium can be designed in such a way that, when the medium is used, it carries out a method according to the invention in a processing unit of the inventive system. Examples of an electronically readable medium are a DVD, a magnetic tape or a USB stick on which electronically readable control information, in particular software, is stored. If this control information is read from the medium and stored in a control unit and/or processing unit of the inventive system, all embodiments of the inventive method and the inventive system described above can be carried out.

In sum, the inventive methods and inventive systems according to the present invention create diagnostic and therapeutic added value for the dentist.

### BRIEF DESCRIPTION OF THE DRAWINGS

In the following description, the present invention will be explained in more detail with reference to the exemplary embodiments and with reference to the drawings, wherein
- Fig. 1: shows a system according to an embodiment of the present invention,
- Fig. 2: shows a practitioner using a scanning device of a system according to an embodiment of the invention,
- Fig. 3: shows a magnetic resonance image acquired by a system according to an embodiment of the invention,
- Fig. 4: shows a flowchart of a computer-implemented method according to an embodiment of the invention,
- Fig. 5: shows a designed dental aligner and an output of a system according to an embodiment of the present invention,
- Fig. 6: shows an initial schedule for the exchange of the designed dental aligners of the initial dental aligner treatment plan proposal according to an embodiment of the present invention,
- Fig. 7: shows a flowchart of a computer-implemented method according to an embodiment of the invention.

### DETAILED DESCRIPTION OF THE EMBODIMENTS

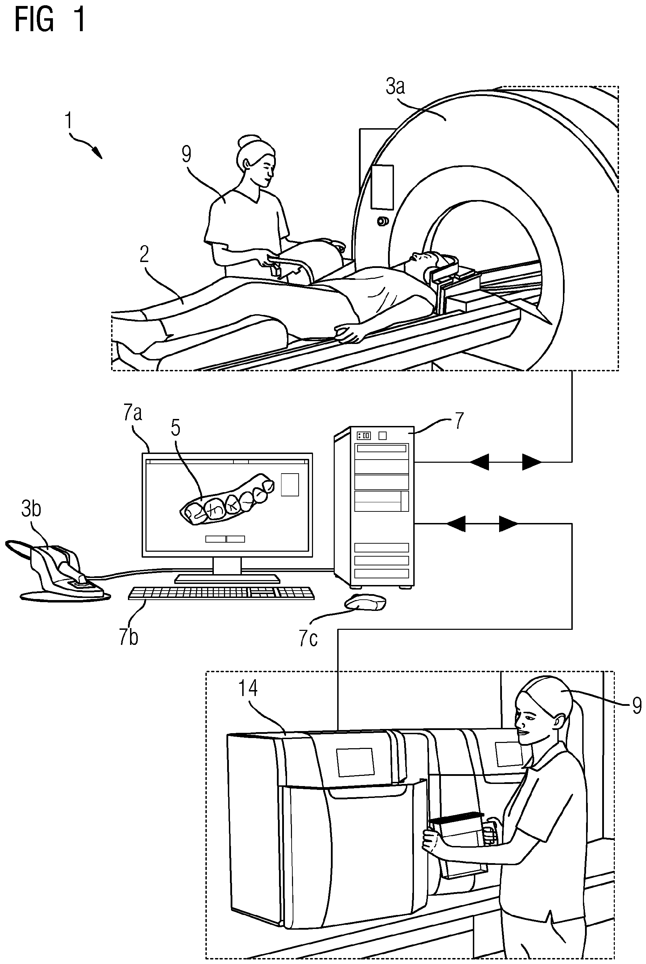

In the following description, the system 1 and the associated computer-implemented method for managing a dental aligner treatment of a patient 2 according to the present invention will be explained in more detail. The computer-implemented method can be carried out on the system 1 as schematically shown in an embodiment in Fig. 1.

The present invention also comprises an associated computer program product having computer-readable codes for respectively implementing the computer-implemented method. The computer program product may be provided on computer readable storage media (not shown) accessible to the system 1. In the following description, the system 1 will be explained in more detail.

As shown in Fig 1, the system 1 comprises at least one acquisition device 3 such as a data interface, a magnetic resonance device 3a and/or a scanning device 3b, particularly an intraoral scanning device. In a preferred embodiment, the system 1 comprises a data interface (not shown) connected to the magnetic resonance device 3a and/or the scanning device 3b via a suitable signal connection. The data interface may be configured to acquire magnetic resonance data 4 from the magnetic resonance device 3a and/or impression data 5 from the scanning device 3b via the signal connection.

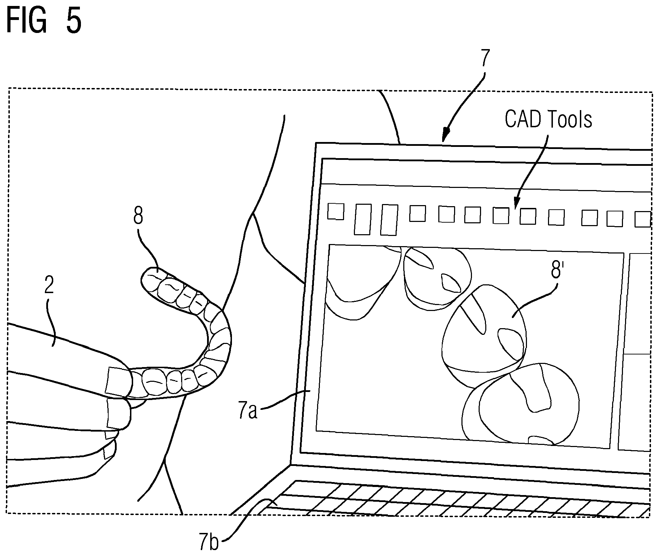

The system 1 may further comprise a manufacturing apparatus 14 configured to manufacture one or more dental aligners 8 (see Fig. 5). Preferably, the manufacturing apparatus 14 comprises a 3D printing unit and a post-processing unit. According to an embodiment, the manufacturing apparatus 14 is an additive manufacturing apparatus configured to manufacture a dental aligner 8 from a photocurable resin. However, the manufacturing apparatus 14 may also represent a milling unit or grinding unit configured to manufacture the dental aligner 8 via removal of material. The magnetic resonance device 3a, the scanning device 3b, and the manufacturing apparatus 14 may be connected to the computing device 7 via any suitable signal connection.

The computing device 7 may be embodied as a computer, a desktop PC, or a workstation comprising one or more processing units. Preferably, a user interface, e. g. comprising a display unit 7a, and input means such as a keyboard 7b, a mouse 7c, or the like, is connected to the computing device 7. The system 1 can be arranged in a medical facility, such as a dental practice. The system 1 can be at least partially configured as an IoT system with cloud computing, wherein components of the system 1 can be arranged at different remote locations connected via a local area network, internet or the like. The components of the system 1 may correspond to the computing device 7, the acquisition device 3, a magnetic resonance device 3a, a scanning device 3b, and/or a manufacturing apparatus 14.

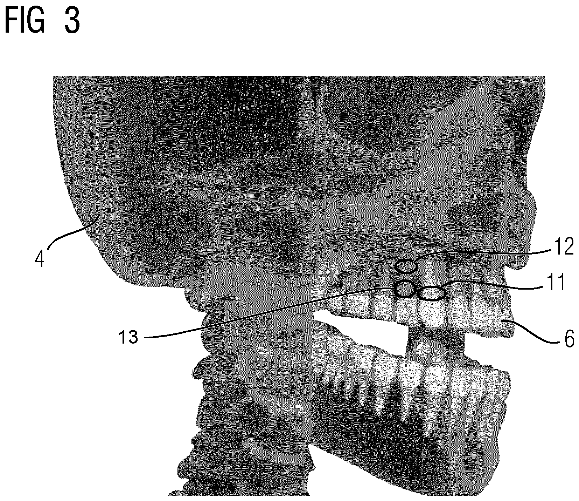

As shown in Fig. 1, the magnetic resonance device 3a is used to acquire magnetic resonance data 4. Preferably, the magnetic resonance data comprises one or more magnetic resonance images of the maxillofacial region of the patient 2. A magnetic resonance image 4 of the maxillofacial region of a patient 2 is shown in Fig. 3. Further details of the magnetic resonance image 4 will be explained below.

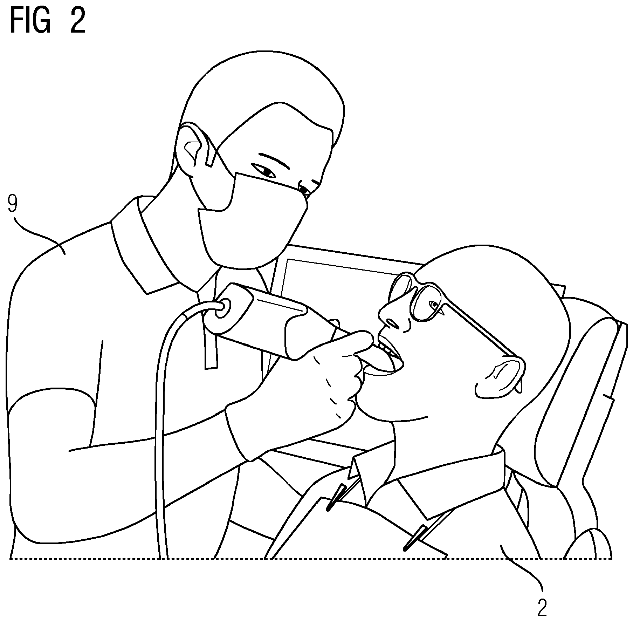

The scanning device 3b is used to acquire impression data 5 of at least some of the teeth 6 of the patient 2, preferably of the entire dentition of the patient 2. As shown in Fig. 2, the scanning device 3b can be configured to generate the impression data 5 by scanning the intraoral cavity and/or the dentition of the patient 2. It is also conceivable that the scanning device 3b is configured to generate the impression data 5 by scanning an impression material (not shown) which has been applied to the teeth 6 of the patient 2. The scanning device 3b may comprise or consist of one or more 2D cameras, a 3D camera, and/or an infrared camera.

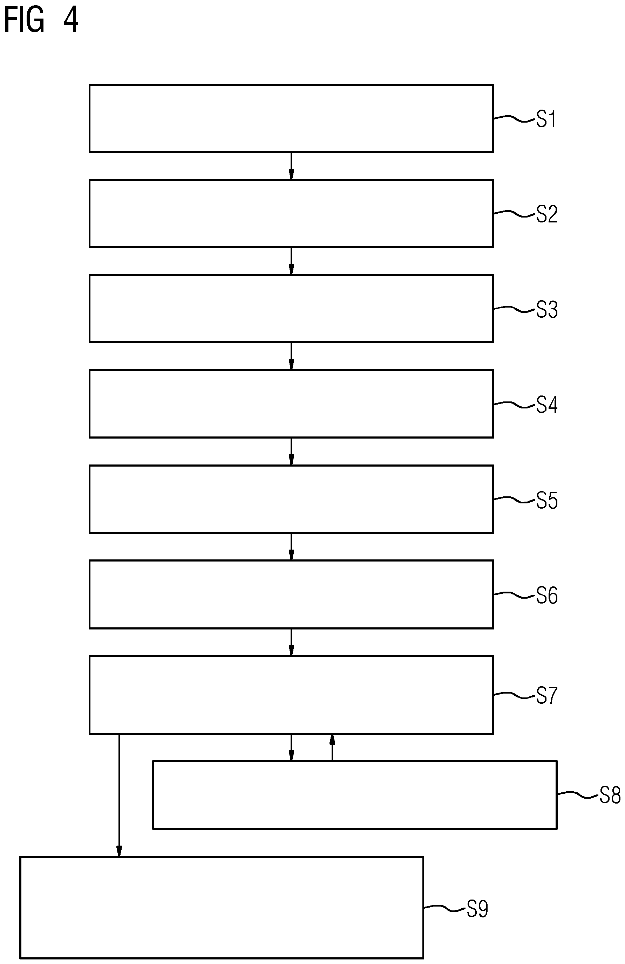

The computing device 7 may be configured to carry out a computer-implemented method according to the embodiment illustrated in Fig. 4.

In the step S1, the magnetic resonance device 3a is used to acquire magnetic resonance data 4 of the maxillofacial region of the patient 2. Preferably, the magnetic resonance data 4 comprises one or more magnetic resonance images 4 of the maxillofacial region of the patient 2. The system 1 acquires the magnetic resonance data 4 from the magnetic resonance device 3a. For that purpose, the computing device 7 may be connected to the magnetic resonance device 3a via a suitable signal connection. It is also conceivable that the computing device 7 comprises a dedicated data interface configured to receive the magnetic resonance data 4 from the magnetic resonance device 3a. The acquisition device 3 may also acquire the magnetic resonance data 4 from a databank storing the magnetic resonance data 4. The magnetic resonance data 4 can be generated before, during and/or after the dental aligner treatment.

In the step S2, the scanning device 3b is used by a practitioner 9 to generate the impression data 5 by scanning the oral cavity or dentition of the patient 2 and/or by scanning an impression material which has been applied to the teeth 6 of the patient 2. The system 1 acquires the impression data 5 from the scanning device 3b. The computing device 7 may be connected to the scanning device 3b via a suitable signal connection. It is also conceivable that the computing device 7 comprises a dedicated data interface configured to receive the impression data 5 from the scanning device 3b. The acquisition device 3 may also be configured to acquire the impression data 5 from a data storage storing the impression data 5. The impression data 5 can be generated before, during and/or after the dental aligner treatment.

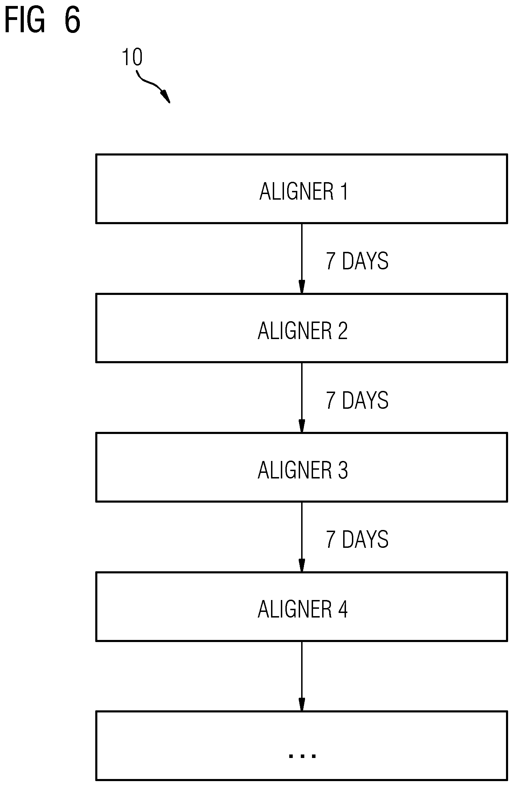

In the step S3, the computing device 7 determines treatment information in dependence of the impression data 5. The treatment information comprises a design of one or more dental aligners 8' and/or an instruction for designing one or more dental aligners 8 to be used during the dental aligner treatment of the patient 2. The treatment information may further comprise an initial schedule 10 for the exchange of the designed dental aligners 8 during the dental aligner treatment of the patient 2. The design of the one or more dental aligners 8' and/or the initial schedule 10 for the exchange of the designed dental aligners 8 during the dental aligner treatment of the patient 2 form a part of the initial dental aligner treatment plan proposal. An example of an initial schedule (10) is show in Fig 6.

In step S4, the computing device 7 outputs the initial dental aligner treatment plan proposal. Preferably, the initial dental aligner treatment plan proposal is output to a practitioner 9 via a display unit 7a connected to the computing device 7. However, the initial dental aligner treatment plan proposal may also be stored in a data storage and/or transmitted to a manufacturing apparatus 14.

In step S5, the computing device 7 analyses the magnetic resonance data 4 and determines a condition of the maxillofacial region of the patient 2.

For example, one or more sections of the maxillofacial region of the patient 2 may be classified as "healthy" or "pathological". It is also conceivable that one or more sections of the maxillofacial region of the patient 2 are classified as being "compatible" or "incompatible" with the dental aligner treatment according to the initial dental aligner treatment plan proposal. Particularly, the computing device 7 may be configured to determine whether the maxillofacial region of the patient 2 allows for a dental aligner treatment according to the initial dental aligner treatment plan proposal or if modifications to the initial dental aligner treatment plan proposal are required.

Optionally, the computing device 7 is configured to determine a pathology in the maxillofacial region of the patient 2 in dependence of the magnetic resonance data 4 and propose a modification to the initial dental aligner treatment plan proposal in dependence of the determined pathology of the maxillofacial region of the patient 2. For example, an image processing unit of the computing device 7 may be configured to analyse the magnetic resonance data 4 and detect a pathology in the maxillofacial region of the patient 2. The computing device 7 may be configured to detect pathologies such as inflammation 11, particularly inflammations at a tooth root tip, bone resorption 12, tooth root resorption 13, and/or a change in a pulp or bone perfusion.

According to an embodiment, the computing device 7 is configured to determine an information on the pathology in dependence of the magnetic resonance data 4. For example, the information may comprise a shape, a dimension, a mass, a type, a location and/or a volume of a pathological tissue. The information about the pathology may be acquired from one or more magnetic resonance images 4, e. g. by processing the one or more magnetic resonance images 4 via a pattern recognition algorithm and/or a dedicated image processing algorithm. According to an embodiment, the computing device 7, particularly a processing unit of the computing device 7, comprises an algorithm configured to detect a pathological tissue in the magnetic resonance data 4 of the maxillofacial region of the patient 2. For example, the algorithm may comprise an AI algorithm, such as an artificial neuronal network, a Bayesian network, an AI classifier, or the like, configured to detect the pathological tissue. It is also conceivable that the processing unit is configured to detect different tissues or organ structures via segmentation.

According to an embodiment, the computing device 7 is configured to determine a deviation from an expected course of the dental aligner treatment. Particularly, the computing device 7 may be configured to determine a modification to the initial dental aligner treatment plan proposal in dependence of the deviation from the expected course of the dental aligner treatment. For example, a progress of the dental aligner treatment may not be in accordance with the dental aligner treatment plan proposal or the modified dental aligner treatment plan proposal. In such a case, an arrangement of teeth may deviate from an expected arrangement of teeth according to the dental aligner treatment plan proposal at a certain point in time during the dental aligner treatment. A deviation from the expected course of the dental aligner treatment may be due to a pathology in the maxillofacial region of the patient 2.

According to an embodiment of the system 1, the computing device 7 is configured to analyse the impression data 5 to detect a pathology in the maxillofacial region of the patient 2 and propose a modification to the initial dental aligner treatment plan proposal in dependence of the determined pathology of the maxillofacial region of the patient 2.

In a further embodiment, the computing device 7 is configured to determine a deviation between an arrangement of teeth according to the initial dental aligner treatment plan proposal and a physiologically appropriate arrangement of teeth in dependence of the magnetic resonance data 4, and propose a modification to the initial dental aligner treatment plan proposal in dependence of the determined deviation. Preferably, the deviation between the arrangement of teeth according to the initial dental aligner treatment plan proposal and the physiologically appropriate arrangement of teeth is determined in the step S5 of the inventive method.

In a preferred embodiment, the magnetic resonance data 4 comprises magnetic resonance data of a temporomandibular joint of the patient 2. The computing device 7 may comprise a motion simulator configured to simulate a motion of the temporomandibular joint of the patient 2 in dependence of the magnetic resonance data 4 of the maxillofacial region of the patient.

In the optional step S6, the computing device 7 analyses the magnetic resonance data 4 to detect a deviation from the expected course of the dental aligner treatment. The computing device 7 can detect said pathologies and said deviations additionally by analysing the impression data 5 generated during the treatment. According to an embodiment, the computing device 7 is adapted to analyse the impression data 5 to detect a pathology in the maxillofacial region of the patient 2 and propose a modification to the initial dental aligner treatment plan proposal in dependence of the determined pathology of the maxillofacial region of the patient 2.

In the step S7, the computing device 7 proposes a modification to the initial aligner treatment plan proposal based on the determined condition of the maxillofacial region of the patient 2.

In the step S8, the computing device 7 outputs the proposed modification to the initial aligner treatment plan proposal. Preferably, the proposed modification to the initial aligner treatment plan proposal is output to the practitioner 9, for example via the display unit 7a.

According to an embodiment, the proposed modification to the initial aligner treatment plan proposal comprises an indication such as a heat map and/or a symbol indicative of an anatomical region for which the treatment should be adapted. The practitioner 9 may approve or digitally revise or edit such modifications by using the CAD tools of the system 1. The outcome of the modifications can be also tested by a second magnetic resonance examination, potentially resulting in another modification of the treatment plan.

According to an embodiment, the computing device 7 is configured to acquire further magnetic resonance data of the maxillofacial region of the patient 2, determine further treatment information in dependence of the further magnetic resonance data, and determine a further dental aligner treatment plan proposal, wherein the further dental aligner treatment plan proposal comprises the further treatment information. The further magnetic resonance data may be acquired at any time during the dental aligner treatment. Preferably, the further magnetic resonance data is acquired via an acquisition device 3, e. g. a magnetic resonance device 3a, a data interface, or the like. The further dental aligner treatment plan proposal may correspond to a modified dental aligner treatment plan proposal. Likewise, the further treatment information may correspond to modified treatment information. The further dental aligner treatment plan proposal is output to a data storage and/or the practitioner 9 according to an embodiment described above.

In an optional step S9, the computing device 7 outputs the proposed (or approved or edited) modification to the design of the one or more dental aligners 8' to the manufacturing apparatus 14, e. g. for additively manufacturing the modified dental aligners.

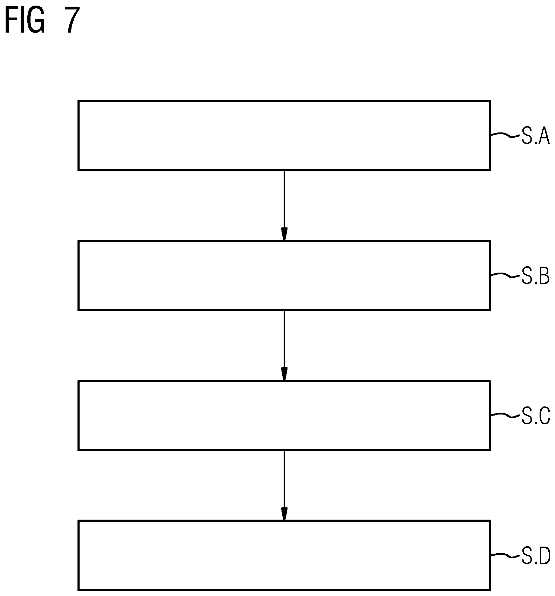

Fig. 7 shows a further flowchart of an inventive computer-implemented method.

In the step S.A, the acquisition device 3 acquires magnetic resonance data 4 of a maxillofacial region of the patient 2 and impression data 5 of a plurality of teeth 6 of the patient 2. The acquisition device 3 may be embodied in accordance with an embodiment described above. In a preferred embodiment, the acquisition device 3 is a magnetic resonance device 3a.

According to the step S.B, the computing device 7 processes the magnetic resonance data 4 and determines a condition of the maxillofacial region of the patient 2. The condition of the maxillofacial region of the patient 2 may be determined according to an embodiment described above.

In the step S.C, the computing device 7 determines treatment information in dependence of the impression data 5 and the magnetic resonance data 4. The treatment information comprises a design of one or more dental aligners 8' (see Fig. 5) to be used during the dental aligner treatment of the patient 2 and/or an initial schedule 10 (see Fig. 6) for an exchange of the designed dental aligners 8 during the dental aligner treatment of the patient 2.

In the step S.D, a dental aligner treatment plan proposal comprising the treatment information is output. Preferably, the dental aligner treatment plan proposal is output to a practitioner according via a display 7a as shown in Fig. 1. The dental aligner treatment plan proposal is determined in dependence of information derived from the impression data 5 and the magnetic resonance data 4. Thus, the dental aligner plan proposal may differ from an initial dental aligner treatment plan proposal determined in dependence of the impression data 5 in accordance with the inventive method described with respect to Fig. 4.

The steps of the inventive method according to Fig. 7 may be carried out via an inventive system 1 as depicted in Fig. 1. The inventive method according to Fig. 7 may include any of the method steps of the embodiment of the inventive method shown in the flowchart depicted Fig. 4.

In the following description, the above-described steps of a computer-implemented method according to the invention will be explained in more detail.

Information on the patient 2 resulting and/or extracted from magnetic resonance data 4 is used as an input for the modification of the design of the dental aligners 8 to improve or optimize the dental aligner treatment result with dental aligners 8 individually per patient and treatment. However, the information on the patient 2 resulting and/or extracted from magnetic resonance data 4 may also be used directly as an input for determining a dental aligner treatment plan proposal.

Preferably, the information on the patient 2 resulting and/or extracted from the magnetic resonance data 4 comprises at least one of the following items:
- an information on a dental tissue state and composition, such as a bone density,
- an information on a patient anatomy (e. g. a location and/or orientation of teeth 6and/or orthodontic anatomical points such as nasion, sella, etc.), including angles and/or distances between the anatomical points),

- an information on a relation between anatomical structures, such as a location of a root of a tooth 6 within the bone,
- an information on a local inflammation (11) (e. g. periodontal or endodontic),
- an information on further functional parameters acquired via native or contrast enhanced contrast mechanisms, e. g. such as pulp or bone perfusion, and/or
- longitudinal data, e. g. based on a comparison of information at different timepoints during the dental aligner treatment (e. g. an information on a speed of movement of teeth).

The information on the patient 2 may be extracted either automatically from the original magnetic resonance data 4 or manually via a user interface. For example, anatomical markers, such as a tip of a tooth root, a tip of a tooth, a geometric centre of a pulp, or the like, may be manually identified in a magnetic resonance image 4. It is also conceivable that a distance between anatomical markers, and/or between an anatomical marker and air, is manually measured in a magnetic resonance image 4. Furthermore, the information on the patient 2 may be extracted by visually inspecting a magnetic resonance image 4.

The impression data 5 and the magnetic resonance data 4 are preferably registered. A registration of the magnetic resonance data 4 and the impression data 5 may comprise rescaling, rotating, and/or translating a magnetic resonance image 4 and/or a 3D scan acquired via the scanning device 3b within a common space. The registration may be based on a correlation function, a correspondence of control points, a global and/or local transformation, a pattern recognition, a radial basis function, a Fourier transform, or the like. The registration process can be complemented using optical and/or magnetic markers, and/or orientation points. Furthermore, registering the impression data 5 with the magnetic resonance data 4 may comprise using a model, particularly a model of the maxillofacial region. The registering of the impression data 5 with the magnetic resonance data 4 may also be based on or carried out via an AI algorithm.

The registration of the magnetic resonance data 4 and the impression data 5 can be achieved automatically by minimizing a distance between point clouds of the impression data 5 and the magnetic resonance data 4. Also, the registration can be achieved by segmenting corresponding anatomical features or anatomical markers. Thereby a location of a pathology in the magnetic resonance data 4 can be related to a location of the pathology in a 3D space of the impression data 5, but also the dental aligner 8.

The information from the registration of the magnetic resonance data 4 and the impression data 5 may be used to determine the condition of the maxillofacial region of the patient 2. Based on the condition of the maxillofacial region of the patient 2, a "default" dental aligner design and/or an initial treatment plan proposal may be modified. It is also conceivable that the dental aligner treatment plan proposal is determined in dependence of the condition of the maxillofacial region of the patient 2.

For example, the condition of the maxillofacial region of the patient 2 may be used to correct or adapt:
- a speed of movement of individual teeth 6 with respect to a "default" case by extrapolation of the information between different timepoints,
- a speed of movement of individual teeth 6 with respect to a "default" case by considering inflammations in the teeth 6 caused by movement,
- a speed of movement of individual teeth 6 due to differences in bone density (e. g. a difference in bone density between the patient 2 and a reference or "default" patient, but also between individual teeth of the patient 2),

- an initial dental aligner treatment plan proposal considering relative and/or absolute positions of anatomical landmarks over time with respect to "default" position changes,
- a speed of movement of individual teeth 6 considering differences in pulp or bone perfusion (e. g. a difference between the patient 2 and a reference or "default" patient, or a difference between individual teeth).

However, the condition of the maxillofacial region of the patient 2 may also be used, for example, to determine:
- a speed of movement of individual teeth 6,
- a speed of movement of individual teeth 6 considering inflammations in the teeth 6 caused by movement,
- a speed of movement of individual teeth 6 considering differences in bone density (e. g. a difference in bone density between the patient 2 and a reference or "default" patient, but also between individual teeth of the patient 2),
- a dental aligner treatment plan proposal considering relative and/or absolute positions of anatomical markers over time,
- a speed of movement of individual teeth 6 considering differences in pulp or bone perfusion (e. g. a difference between the patient 2 and a reference or "default" patient, or a difference between individual teeth).

The modifications described above may be achieved or implemented, for example, by changing a shape and/or a dimension of a section of one or more dental aligners 8. The modified dental aligners 8 may expose the plurality of teeth 6 of the patient 2 to an altered or modified stress pattern. Particularly, the modifications to the design of the one or more dental aligners 8 and/or the initial dental aligner treatment plan proposal according to an embodiment described above may favourably improve or optimize a result of the dental aligner treatment and/or reduce a risk of damage to the patient 2. A modification to the design of the one or more dental aligners can be made either manually, e. g. by a user using a CAD tool, or automatically.

By additionally acquiring impression data 5 at the same time as magnetic resonance data 4 and extracting information from the impression data 5, or from the combination of magnetic resonance data 4 and impression data 5, and by using this additional information (e. g. by combining tooth surface information from a 3D scan and dental root information from a magnetic resonance image) as an additional input to the aligner design, the treatment result can be optimized even further.

Magnetic resonance data 4 may be acquired via a first magnetic resonance examination after a first period of treatment, e. g. to monitor treatment success and adverse effects, such as inflammation. After a second period of treatment, preferably in dependence of the first magnetic resonance examination, a second magnetic resonance examination may be performed to test or check an effect of the adopted/modified treatment (e. g. the initial dental aligner treatment plan proposal). The test may include a comparison of a level and/or extent of inflammation and/or a speed of progression of bone loss. A further adoption/modification of the treatment plan may be implemented and checked after a further treatment period. This procedure may be repeated until any adverse effects of the treatment are sufficiently diminished.

The scope of the invention includes taking one or more scans or evaluations with one or more different modalities over time (e. g. digital volume tomography), as long as at least one of the scans is a magnetic resonance examination.

According to a preferred embodiment of the invention, magnetic resonance data 4 is acquired before a start of the dental aligner treatment and further magnetic resonance data is acquired during the dental aligner treatment. The computing device 7 may be configured to carry out an automated analysis of tooth trajectories over time, an analysis of a tooth root position with respect to a cortical bone, and/or an analysis of early signs of inflammation, but also information regarding a temporomandibular joint. The design of the one or more dental aligners may be corrected to optimize a speed of the dental aligner treatment without harming the patient.

The embodiments described above are to be recognized as examples. It is to be understood that individual embodiments may be extended by or combined with features of other embodiments if not stated otherwise. Particularly, the sequence of the steps of the inventive method is to be understood as an example. Individual steps may be carried out in a different order and/or overlap partially or completely in time.

## Claims

1. A system (1) for managing a dental aligner treatment of a patient (2), the system (1) comprising:
- an acquisition device (3; 3a; 3b) adapted to acquire magnetic resonance data (4) of a maxillofacial region of the patient (2), and impression data (5) of a plurality of teeth (6) of the patient (2); and
- a computing device (7) adapted to:
• determine treatment information in dependence of the impression data (5), wherein the treatment information comprises a design of one or more dental aligners (8) to be used during the dental aligner treatment of the patient (2) and/or an initial schedule (10) for an exchange of the designed dental aligners (8) during the dental aligner treatment of the patient (2),
• output an initial dental aligner treatment plan proposal comprising the treatment information,
• analyse the magnetic resonance data (4) and determine a condition of the maxillofacial region of the patient (2), and
• propose a modification to the initial dental aligner treatment plan proposal in dependence of the determined condition of the maxillofacial region of the patient (2).

2. The system (1) according to claim 1, **characterized in that** the computing device (7) is adapted to output the proposed modification to a practitioner (9).

3. The system (1) according to one of the claims 1 or 2, **characterized in that** the proposed modification to the initial dental aligner treatment plan proposal comprises a heat map and/or a symbol indicative of an anatomical region for which the dental aligner treatment should be adapted.

4. The system (1) according to one of the preceding claims, wherein the treatment information comprises a design of one or more dental aligners (8) to be used during the dental aligner treatment of the patient (2) and wherein the proposed modification to the initial dental aligner treatment plan proposal comprises a modification to the design of the one or more dental aligners (8).

5. The system (1) according to claim 4, **characterized in that** the computing device (7) is adapted to output the proposed modification to the design of the one or more dental aligners (8) to a manufacturing apparatus (14).

6. The system (1) according to one of the preceding claims, **characterized by** comprising a scanning device (3b) configured to generate the impression data (5) by scanning an oral cavity of the patient (2) and/or an impression material which has been applied to the teeth (6) of the patient (2).

7. The system (1) according to one of the preceding claims, **characterized in that** the computing device (7) is adapted to
• analyse the impression data (5) to detect a pathology in the maxillofacial region of the patient (2) and
• propose a modification to the initial dental aligner treatment plan proposal in dependence of the determined pathology of the maxillofacial region of the patient (2).

8. The system according to any one of the preceding claims, **characterized in that** the acquisition device (3; 3a) is configured to acquire further magnetic resonance data of the maxillofacial region of the patient (2), and
the computing device (7) is configured to:
• determine further treatment information in dependence of the further magnetic resonance data, and
• output the further treatment information.

9. The system according to one of the preceding claims, wherein the computing device (7) is configured to:
• determine a pathology in the maxillofacial region of the patient (2) in dependence of the magnetic resonance data (4), and
• propose a modification to the initial dental aligner treatment plan proposal in dependence of the determined pathology of the maxillofacial region of the patient (2).

10. The system according to claim 9, wherein the determined pathology in the maxillofacial region of the patient (2) comprises an inflammation (11), a bone resorption (12), a tooth root resorption (13), a change in a pulp or bone perfusion, and/or a deviation from an expected course of the dental aligner treatment.

11. The system (1) according to one of the preceding claims, wherein the computing device (7) is configured to:
• determine a deviation between an arrangement of teeth (6) according to the initial dental aligner treatment plan proposal and a physiologically appropriate arrangement of teeth (6) in dependence of the magnetic resonance data (4), and
• propose a modification to the initial dental aligner treatment plan proposal in dependence of the determined deviation.

12. The system (1) according to claim 11, wherein the magnetic resonance data (4) comprises magnetic resonance data of a temporomandibular joint of the patient (2), and wherein the computing device (7) comprises a motion simulator configured to simulate a motion of a temporomandibular joint in dependence of the magnetic resonance data (4).

13. A computer-implemented method for managing a dental aligner treatment of a patient (2) using a system (1) according to one of the preceding claims, comprising the steps:
• acquiring magnetic resonance data (4) of a maxillofacial region of the patient (2) and impression data (5) of a plurality of teeth (6) of the patient (2) via an acquisition device (3, 3a, 3b),
• determining treatment information in dependence of the impression data (5) via a computing device (7), wherein the treatment information comprises a design of one or more dental aligners (8) to be used during the dental aligner treatment of the patient (2) and/or an initial schedule (10) for the exchange of the designed dental aligners (6) during the dental aligner treatment of the patient (2),
• outputting an initial dental aligner treatment plan proposal comprising the treatment information,
• analysing the magnetic resonance data (4) and determine a condition of the maxillofacial region of the patient (2), and
• proposing a modification to the initial dental aligner treatment plan proposal in dependence of the determined condition of the maxillofacial region of the patient (2).

14. A system (1) for managing a dental aligner treatment of a patient (2), the system (1) comprising:
- an acquisition device (3; 3a; 3b) adapted to acquire magnetic resonance data (4) of a maxillofacial region of the patient (2), and impression data (5) of a plurality of teeth (6) of the patient (2); and
- a computing device (7) configured to:
• analyse the magnetic resonance data (4) and determine a condition of the maxillofacial region of the patient (2),
• determine treatment information in dependence of the impression data (5) and the determined condition of the maxillofacial region of the patient (2), wherein the treatment information comprises a design of one or more dental aligners (8) to be used during the dental aligner treatment of the patient (2) and/or an initial schedule (10) for an exchange of the designed dental aligners (8) during the dental aligner treatment of the patient (2), and
• output a dental aligner treatment plan proposal comprising the treatment information.

15. A computer-implemented method for managing a dental aligner treatment of a patient (2) using a system (1) according to claim 14, comprising the steps:
• acquiring magnetic resonance data (4) of a maxillofacial region of the patient (2) and impression data (5) of a plurality of teeth (6) of the patient (2) via an acquisition device (3, 3a, 3b),
• analysing the magnetic resonance data (4) and determining a condition of the maxillofacial region of the patient (2) via a computing device (7),
• determining treatment information in dependence of the impression data (5) and the determined condition of the maxillofacial region of the patient (2) via the computing device (7), wherein the treatment information comprises a design of one or more dental aligners (8) to be used during the dental aligner treatment of the patient (2) and/or an initial schedule (10) for an exchange of the designed dental aligners (8) during the dental aligner treatment of the patient (2), and
• outputting a dental aligner treatment plan proposal comprising the treatment information.

16. A computer program product configured to be loaded into a memory of a programmable processing unit of a system (1) according to one of the claims 1 to 12 or 14, comprising program code means to perform a method according to claim 13 or 15 when the computer program product is executed in the processing unit of the system (1).
